# EUROPEAN PATENT APPLICATION

(11) **EP 2 266 970 A1**
(43) Date of publication of application: **29.12.2010**
(21) Application number: 10179273.7
(22) Date of filing: 16.06.2008
(51) Int. Cl.: C07D 401/06, C07D 403/06, C07D 405/14, C07D 409/14, A61P 43/00, A61K 31/4025

(54) **Substituted aromatic heterocyclic compounds as fungicides**

(30) Priority: 18.06.2007 GB 0711776
(62) Divisional of application: 08759253.1
(71) Applicant: Syngenta Participations AG, 4058 Basel (CH)
(72) Inventor: Ackermann, Peter, 4148, Pfeffingen (CH); Bobbio, Carla, 4332, Stein (CH); Corsi, Camilla, 4332, Stein (CH); Mcginley, Ann Monica M., 4125, Riehen (CH); Ehrenfreund, Josef, 4123, Allschwil (CH)
(74) Representative: Herrmann, Jörg

(57) **Abstract**

The present invention relates to compounds of formula I 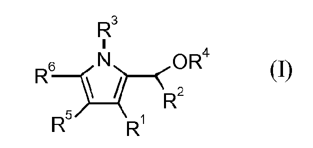 wherein R¹, R², R³, R⁴, R⁵ and R⁶ are as defined in claim 1 or a salt or N-oxide thereof and their use in methods for the control and/or prevention of fungal infection, particularly in plants.

## Description

The present invention relates to novel substituted pyrrole-containing compounds, and their use in methods for the control and/or prevention of fungal infection, particularly in plants.

The incidence of serious fungal infections, either systemic or topical, continues to increase for plants, animals, and humans. Many fungi are common in the environment and not harmful to plants or mammals. However, some fungi can produce disease in plants, humans and/or animals.

Fungicides are compounds, of natural or synthetic origin, which act to protect plants against damage caused by fungi, including oomycetes. Current methods of agriculture rely heavily on the use of fungicides. In fact, some crops cannot be grown usefully without the use of fungicides. Using fungicides allows a grower to increase the yield of the crop and consequently, increase the value of the crop. Numerous fungicidal agents have been developed. However, the treatment of fungal infestations and infections continues to be a major problem. Furthermore, fungicide and antifungal drug resistance has become a serious problem, rendering these agents ineffective for some agricultural and therapeutic uses. As such, a need exists for the development of new fungicidal and antifungal compounds.

Accordingly, the present invention provides a compound of formula I: 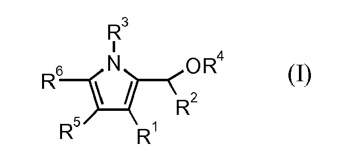 wherein:
R¹ and R³ are, independently, hydrogen, or optionally substituted alkyl, alkenyl, alkynyl, heterocyclyl, trialkylsilyl, arylalkyl, aryloxyalkyl, arylthioalkyl, aryl or heteroaryl, with the proviso that they are not both hydrogen;
R² is optionally substituted alkyl, alkenyl, alkynyl, heterocyclyl, arylalkyl, aryl or heteroaryl;
R⁴ is H or a group which under biological conditions can be cleaved to form an alcohol at this position (i.e. -OR⁴ is -OH). Examples of such groups are acyl, haloacyl, alkoxycarbonyl, aryloxycarbonyl, alkylaminocarbonyl or dialkylaminocarbonyl groups.
R⁵ and R⁶ are, independently, hydrogen, cyano, halogen or optionally substituted alkyl, alkenyl, alkynyl, heterocyclyl, alkoxy, alkoxycarbonyl, alkylthio, trialkylsilyl, arylalkyl, aryloxyalkyl, arylthioalkyl, aryl or heteroaryl;
or a salt or N-oxide thereof.

Unless otherwise stated, the following terms used in the specification and claims have the meanings given below:
"Alkyl" means a linear saturated monovalent hydrocarbon radical of one to eight carbon atoms or a branched saturated monovalent hydrocarbon radical of three to eight carbon atoms, e.g. methyl, ethyl, *n*-propyl, *iso*-propyl, *n*-butyl, *sec*-butyl, *iso-*butyl, *tert*-butyl, *n*-pentyl, *iso*-amyl, *n*-hexyl and the like. It is noted that this definition applies both when the term is used alone and when it is used as part of a compound term, such as "haloalkyl" and similar terms. Preferably, linear alkyl groups contain one to six carbon atoms, more preferably one to four carbon atoms and most preferably are selected from methyl, ethyl or *n*-propyl. Preferably, branched alkyl groups contain three to six carbon atoms and more preferably are selected from *iso*-propyl (1-methylethyl), *sec*-butyl (1-methylpropyl), *iso*-butyl (2-methylpropyl), *tert*-butyl (1,1-dimethylethyl) or *iso*-amyl (3-methylbutyl).
"Alkenyl" means a linear monovalent saturated hydrocarbon radical of two to eight carbon atoms, or a branched monovalent hydrocarbon radical of three to eight carbon atoms containing at least one double bond, e.g. ethenyl, propenyl and the like. Where appropriate, an alkenyl group can be of either the (E)- or (Z)-configuration. Preferably, linear alkenyl groups contain two to six carbon atoms and more preferably are selected from ethenyl, prop-1-enyl, prop-2-enyl, prop-1,2-dienyl, but-1-enyl, but-2-enyl, but-3-enyl, but-1,2-dienyl and but-1,3-dienyl. Preferably, branched alkenyl groups contain three to six carbon atoms and more preferably are selected from 1-methylethenyl, 1-methylprop-1-enyl, 1-methylprop-2-enyl, 2-methylprop-1-enyl and 2-methylprop-2-enyl.
"Alkyl" and "alkenyl" groups also encompass cycloalkyl and cycloalkenyl groups, respectively. These are monovalent cyclic hydrocarbon radicals of three to eight ring carbons and, more preferably, three to six ring carbons. Cycloalkyl groups are fully saturated, while cycloalkenyl groups may be mono- or di-unsaturated. Preferably, cycloalkyl groups are selected from cyclopropyl, cyclobutyl, cyclopentyl and cyclohexyl. Preferably, mono-unsaturated cycloalkenyl groups are selected from cyclobutenyl, cyclopentenyl and cyclohexenyl.
"Heterocyclyl" means a cyclic hydrocarbon radical as defined above containing one, two or three ring heteroatoms selected from N, O or S(O)ₙ (where n is an integer from 0 to 2), the remaining ring atoms being carbon where one or two carbon atoms may optionally be replaced by a carbonyl group. Examples of such rings include, but are not limited to, oxirane, oxetane, tetrahydrofuran, tetrahydropyran, 1,3-dioxolane, 1,4-dioxane, aziridine, azetidine, pyrrolidine, piperidine, oxazinane, morpholine, thiomorpholine, imidazolidine, pyrazolidine and piperazine. More preferably, the heterocyclyl group contains three to six ring atoms including one O and/or one N ring atom.
"Alkynyl" means a linear monovalent saturated hydrocarbon radical of two to eight carbon atoms, or a branched monovalent hydrocarbon radical of five to eight carbon atoms, containing at least one triple bond, e.g. ethynyl, propynyl and the like. Preferably, linear alkynyl groups contain two to six carbon atoms and more preferably are selected from ethynyl, prop-1-ynyl, prop-2-ynyl, but-1-ynyl, but-2-ynyl and but-3-ynyl. Preferably, branched alkynyl groups contain four to six carbon atoms and more preferably are selected from 1-methylprop-2-ynyl, 3-methylbut-1-ynyl, 1-methylbut-2-ynyl, 1-methylbut-3-ynyl and 1-methylbut-3-ynyl.
"Alkoxy" means a radical -OR, where R is alkyl, alkenyl or alkynyl. Alkoxy groups include, but are not limited to, methoxy, ethoxy, 1-methylethoxy, propoxy, 1-methylpropoxy and 2-methylpropoxy. Preferably alkoxy means methoxy or ethoxy.
"Alkylthio" means a radical -SR, where R is alkyl, alkenyl or alkynyl. Alkylthio groups include, but are not limited to, methylthio, ethylthio, *tert*-butylthio, hexylthio, and the like.
"Aryl" or "aromatic ring moiety" refers to an aromatic substituent which may be a single ring or multiple rings which are fused together, linked covalently or linked to a common group such as an ethylene or methylene moiety. The aromatic rings may each contain one or more heteroatoms and hence "aryl" encompasses "heteroaryl". Representative examples of aryl include, for example, azulenyl, indanyl, indenyl, naphthyl, phenyl, tetrahydronaphthyl, biphenyl, diphenylmethyl, 2,2-diphenyl-1-ethyl, thienyl, pyridyl, pyrimidinyl and quinoxalyl. "Aryl" means substituted or unsubstituted aryl unless otherwise indicated and hence the aryl moieties may be optionally substituted with one or more of the same or different halogen atoms and/or one or more other groups such as nitro, carboxyl, alkoxy, phenoxy and the like. Additionally, the aryl radicals may be attached to other moieties at any position on the aryl radical which would otherwise be occupied by a hydrogen atom (such as, for example, 2-pyridyl, 3-pyridyl and 4-pyridyl).

In particular, "heteroaryl" means a cyclic, aromatic ring in which one or more carbon atoms have been replaced with heteroatoms. If the heteroaryl group contains more than one heteroatom, the heteroatoms may be the same or different. Examples of heteroaryl groups include pyridyl, pyrimidinyl, imidazolyl, thienyl, furyl, pyrazinyl, pyrrolyl, pyranyl, isobenzofuranyl, chromenyl, xanthenyl, indolyl, isoindolyl, indolizinyl, triazolyl, pyridazinyl, indazolyl, purinyl, quinolizinyl, isoquinolyl, quinolyl, phthalazinyl, naphthyridinyl, quinoxalinyl, isothiazolyl, and benzo[b]thienyl. Preferred heteroaryl groups are five and six membered rings and contain from one to three heteroatoms independently selected from O, N, and S. The heteroaryl group, including the ring carbons and each heteroatom, can be unsubstituted or substituted with from 1 to 4 substituents, as chemically feasible. For example, the heteroatom S may be substituted with one or two oxo groups, which may be shown as =O.
"Halo" or "halogen" means fluoro, chloro, bromo or iodo, preferably chloro or fluoro.
"Haloalkyl" means alkyl as defined above substituted with one or more of the same or different halo atoms. Examples of haloalkyl groups include, but are not limited to chloromethyl, fluoromethyl, difluoromethyl, trifluoromethyl, 2-fluoroethyl, 2-trifluoroethyl, 2-chloro-ethyl, 2-iodoethyl, 3-fluoropropyl, 3-chloropropyl, 2-trifluoro-1-chloroethyl and 1-difluoro-2-difluoro-3-trifluoropropyl.
"Haloalkenyl" means alkenyl as defined above substituted with one or more of the same or different halo atoms. Examples of haloalkenyl groups include, but are not limited to 2-dibromoethenyl, 2-fluoro-2-bromoethenyl, 5-bromopent-3-enyl and 3 dichloroprop-2-enyl.
"Haloalkoxy" means a radical -OR, wherein R is haloalkyl or haloalkenyl.
"Haloalkylthio" means a radical -SR, wherein R is haloalkyl.
"Trialkylsilyl" means the group -Si(R)₃, wherein each R is, independently, an alkyl group as defined above.
"Arylalkyl" means a radical -R^{a}R^{b} where R^{a} is an alkylene or alkenylene group as defined below and R^{b} is an aryl group as defined above.
"Alkylene" means a linear saturated divalent hydrocarbon radical of one to six carbon atoms or a branched saturated divalent hydrocarbon radical of three to six carbon atoms, e.g. methylene, ethylene, propylene, 2-methylpropylene and the like. Preferred alkylene groups are the divalent radicals of the alkyl groups defined above.
"Alkenylene" means a linear divalent hydrocarbon radical of two to six carbon atoms or a branched divalent hydrocarbon radical of three to six carbon atoms, containing at least one double bond, e.g. ethenylene, propenylene and the like. Preferred alkenylene groups are the divalent radicals of the alkenyl groups defined above.
"Aryloxyalkyl" means a radical -R^{a}OR^{b}, wherein R^{a} is an alkylene or alkenylene group and R^{b} is an aryl group as defined above.
"Arylthioalkyl" means a radical -R^{a}SR^{b}, wherein R^{a} is an alkylene or alkenylene group and R^{b} is an aryl group as defined above.
"Acyl" means -C(O)R, wherein R is hydrogen, alkyl, alkenyl, alkynyl, heterocyclyl, aryl or heteroaryl. Examples of acyl groups include formyl, alkylcarbonyl, alkenylcarbonyl and arylcarbonyl groups.
"Haloacyl" means -C(O)R, wherein R is haloalkyl or haloalkenyl.
"Alkoxycarbonyl" means -C(O)OR, wherein R is hydrogen, alkyl, alkenyl, or alkynyl.
"Aryloxycarbonyl" means -C(O)OR, wherein R is aryl.
"Alkylaminocarbonyl" means -C(O)NHR, wherein R is alkyl.
"Dialkylaminocarbonyl" means -C(O)N(R)₂, wherein each R is independently alkyl.
"Cyano" means a -CN group.
"Hydroxy" means an -OH group.
"Nitro" means an -NO₂ group.
"Oxy" means an -O- moiety.
"Thio" as used herein, refers to a -S- moiety.
"Optionally substituted" means substituted by one or more substituents, in particular, one, two, three or four substituents, independently selected from halogen, hydroxyl, cyano, nitro, alkyl, haloalkyl, alkenyl, haloalkenyl, alkynyl, haloalkynyl, heterocyclyl, aryl, heteroaryl, alkoxy, haloalkoxy, alkylthio, haloalkylthio, acyl, alkoxycarbonyl and trialkylsilyl. Preferred optional substituents include halogen (in particular, fluoro, chloro or bromo), cyano, nitro, alkyl (in particular, methyl and ethyl), haloalkyl, alkenyl, haloalkenyl, alkynyl, alkoxy (in particular, methoxy or ethoxy), haloalkoxy, alkylthio, haloalkylthio.

The compounds of formula I may exist in different geometric or optical isomeric forms or in different tautomeric forms. One or more centres of chirality may be present, in which case compounds of the formula I may be present as pure enantiomers, mixtures of enantiomers, pure diastereomers or mixtures of diastereomers. There may be double bonds present in the molecule, such as C=C or C=N bonds, in which case compounds of formula I may exist as single isomers of mixtures of isomers. Centres of tautomerisation may be present. This invention covers all such isomers and tautomers and mixtures thereof in all proportions as well as isotopic forms such as deuterated compounds.

Suitable salts of the compounds of formula I include acid addition salts such as those with an inorganic acid such as hydrochloric, hydrobromic, sulphuric, nitric or phosphoric acid, or an organic carboxylic acid such as oxalic, tartaric, lactic, butyric, toluic, hexanoic or phthalic acid, or a sulphonic acid such as methane, benzene or toluene sulphonic acid. Other examples of organic carboxylic acids include haloacids such as trifluoroacetic acid.

N-oxides are oxidised forms of tertiary amines or oxidised forms of nitrogen containing heteroaromatic compounds. They are described in many books for example in "Heterocyclic N-oxides" by Angelo Albini and Silvio Pietra, CRC Press, Boca Raton, Florida, 1991.

In particularly preferred embodiments of the invention, the preferred groups for R¹ to R⁶, in any combination thereof, are as set out below.

In one embodiment, R¹ and R³ are, independently, hydrogen or optionally substituted alkyl, alkenyl, alkynyl, heterocyclyl or trialkylsilyl.

Alkyl, alkenyl, alkynyl and trialkylsilyl groups are as defined above. In particular, alkyl groups include methyl, ethyl, *iso*-propyl, butyl, *iso*-butyl, iso-amyl and cyclohexyl groups.

In another embodiment, R¹ and R³ are, independently, hydrogen or optionally substituted alkyl, aryloxyalkyl, arylthioalkyl, aryl or heteroaryl.

Aryl groups are as defined above. In particular, aryl groups include benzyl or phenyl groups optionally substituted with one or more (in particular, one or two) of the same or different halogen atoms (for example chloro and fluoro), haloalkyl or haloalkoxy groups. For example, aryl groups include benzyl, phenyl, 2-chlorophenyl, 3-chlorophenyl, 4-chlorophenyl, 2,4-dichlorophenyl, 2-fluorophenyl, 3-flurophenyl, 4-fluorophenyl, 2-fluoro-4-chlorophenyl, 2,4-dichlorophenyl, 2,4-difluorophenyl, 3,5-difluorophenyl, 3-trifluoromethylphenyl, , 4-trifluoromethylphenyl and 4-trifluoromethoxyphenyl.

Heteroaryl groups are as defined above. In particular, heteroaryl groups include 5- or 6- membered heteroaryl rings such as furyl and thienyl rings. These heteroaryl groups may be optionally substituted by one or more (in particular, one or two) of the same of different halogen atoms and in clude, for example, 2-furyl, 3-furyl, 2-thienyl, 3-thienyl, 5-chloro-2-thienyl, or 5-chloro-2-furyl.

In another embodiment, R¹ and R³ are, independently, hydrogen or optionally substituted alkyl, phenyl or 5- or 6-membered heteroaryl.

In one embodiment, R² is optionally substituted heteroaryl. Heteroaryl is as defined above and, in particular, is pyridyl, pyrimidinyl or thiazolyl optionally substituted with one or more (in particular, one or two) of the same or different halogen, alkyl or alkoxy groups. Examples include 2-pyridyl, 3-pyridyl, 4-pyridyl, 3-pyrimidinyl, 5-pyrimidinyl, 4-fluoro-3-pyridyl, 4-methyl-3-pyridyl, 5-methoxy-3-pyridyl, 4-methyl-5-pyrimidinyl, 4-methoxy-5-pyrimidinyl, 2-thiazolyl or 5-thiazolyl.

In one embodiment, R² is optionally substituted pyridyl, pyrimidinyl or thiazolyl.

In one embodiment, R⁴ is H.

In one embodiment, R⁵ and R⁶ are, independently hydrogen, cyano or halogen or optionally substituted alkyl, alkenyl, alkynyl, alkoxy, alkylthio, trialkylsilyl or alkoxycarbonyl. In particular, R⁵ and R⁶ are, independently hydrogen, cyano, halogen, alkyl, alkoxy or alkylthio.

In one embodiment, at least one of R¹, R³, R⁵, and R⁶ is not hydrogen , the other groups, including R² and R⁴ being as defined above.

In one embodiment, R¹ and R³ are, independently, optionally substituted aryl or heteroaryl; R² is optionally substituted heteroaryl; and R⁴, R⁵, and R⁶ are hydrogen.

In another embodiment, R¹ and R³ are, independently, optionally substituted phenyl, thienyl, pyridyl or furyl; R² is optionally substituted pyridyl or pyrimidinyl; and R⁴, R⁵, and R⁶ are hydrogen.

In a preferred embodiment, R¹ is 2-chloro-phenyl, 3-chloro-phenyl, 4-chloro-phenyl, 4-bromo-phenyl, 2-fluoro-phenyl, 4-fluoro-phenyl, 2,4-dichloro-phenyl, 2,4-difluoro-phenyl, 2-fluoro-4-chloro-phenyl, 2-chloro-4-fluoro-phenyl, 2-methyl-phenyl, 4-methyl-phenyl, 2,4-dimethyl-phenyl, 2-methoxy-phenyl, 4-methoxy-phenyl, 3-trifluoromethyl-phenyl, 4-trifluoromethyl-phenyl, 2-chloro-4-methoxy-phenyl, 4-methoxytrifluomethyl-phenyl, 2-methyl-4-chloro-phenyl, 2-chloro-3-pyridyl, 2-thienyl, 3-thienyl or 5-chloro-2-thienyl; and R², R³,R⁴, R⁵ and R⁶ are as described in any embodiment above.

In a most preferred embodiment, R³ is 3-chloro-phenyl; and R¹, R², R⁴, R⁵ and R⁶ are as described in any embodiment above.

In an especially preferred embodiment, R² is 3-pyridyl; and R¹, R³, R⁴, R⁵ and R⁶ are as described in any embodiment above.

In one embodiment, R¹ is optionally substituted alkyl, alkenyl, alkynyl, heterocyclyl, trialkylsilyl, arylalkyl, aryloxyalkyl, arylthioalkyl, aryl or heteroaryl; R³ is hydrogen; and R², R⁴, R⁵ and R⁶ are as described in any embodiment above.

In one embodiment, R¹ is hydrogen; R³ is optionally substituted alkyl, alkenyl, alkynyl, heterocyclyl, trialkylsilyl arylalkyl, aryloxyalkyl, arylthioalkyl, aryl or heteroaryl; and R², R⁴, R⁵ and R⁶ are as described in any embodiment above.

In one embodiment, R¹ is optionally substituted aryloxyalkyl, arylthioalkyl, aryl or heteroaryl; R³ is hydrogen, optionally substituted alkyl, alkenyl, alkynyl, heterocyclyl or trialkylsilyl; and R², R⁴, R⁵ and R⁶ are as described in any embodiment above.

In one embodiment, R¹ is hydrogen, optionally substituted alkyl, alkenyl, alkynyl, heterocyclyl or trialkylsilyl; R³ is optionally substituted aryloxyalkyl, arylthioalkyl, aryl or heteroaryl; and R², R⁴, R⁵ and R⁶ are as described in any embodiment above.

In one embodiment, R¹ is optionally substituted aryl or heteroaryl; R³ is hydrogen or optionally substituted alkyl; and R², R⁴, R⁵ and R⁶ are as described in any embodiment above.

In one embodiment, R¹ is hydrogen or optionally substituted alkyl; R³ is optionally substituted aryl or heteroaryl; and R², R⁴, R⁵ and R⁶ are as described in any embodiment above.

In one embodiment, R¹ is optionally substituted phenyl or 5- or 6-membered heteroaryl; R³ is hydrogen, optionally substituted alkyl, alkenyl, alkynyl or heterocyclyl; and R², R⁴, R⁵ and R⁶ are as described in any embodiment above.

In one embodiment, R¹ is hydrogen, optionally substituted alkyl, alkenyl, alkynyl or heterocyclyl; R³ is optionally substituted phenyl or 5- or 6-membered heteroaryl; and R², R⁴, R⁵ and R⁶ are as described in any embodiment above.

More particularly, compounds for use in the present invention are shown in Table 1 below:

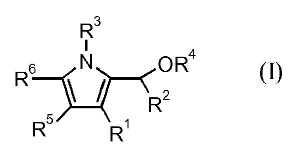

| No | R¹ | R² | R³ | R⁴ | R⁵ | R⁶ |
|---|---|---|---|---|---|---|
| 1 | 4-Cl-Ph | 3-Py | 4-Cl-Ph | H | H | H |
| 2 | 4-Cl-Ph | 3-Py | 4-Cl-Ph | H | H | Me |
| 3 | 4-Cl-Ph | 3-Py | 4-Cl-Ph | C(O)Me | H | H |
| 4 | 4-Cl-Ph | 3-Py | 4-Cl-Ph | H | Me | H |
| 5 | 4-Cl-Ph | 3-Py | 2-Cl-Ph | H | H | H |
| 6 | 4-Cl-Ph | 3-Py | 2-Cl-Ph | H | Et | H |
| 7 | 4-Cl-Ph | 3-Py | 3-Cl-Ph | H | H | H |
| 8 | 4-Cl-Ph | 3-Py | 3-Cl-Ph | H | 4-Cl-Ph | H |
| 9 | 2,4-Cl₂-Ph | 3-Py | 3-Cl-Ph | C(O)Et | H | H |
| 10 | 2,4-Cl₂-Ph | 3-Py | 4-Cl-Ph | H | H | H |
| 11 | 2,4-Cl₂-Ph | 3-Py | 4-Cl-Ph | H | Bu | H |
| 12 | 2,4-Cl₂-Ph | 3-Py | 2-Cl-Ph | H | H | H |
| 13 | 2,4-Cl₂-Ph | 3-Py | 3-Cl-Ph | H | H | H |
| 14 | 4-Cl-Ph | 3-Py | 2,4-Cl₂-Ph | H | H | H |
| 15 | 2-Cl-Ph | 3-Py | 2,4-Cl₂-Ph | H | H | H |
| 16 | 3-Cl-Ph | 3-Py | 2,4-Cl₂-Ph | H | H | H |
| 17 | 4-Cl-Ph | 5-Pyrimi | 4-Cl-Ph | H | H | H |
| 18 | 4-Cl-Ph | 5-Pyrimi | 2-Cl-Ph | H | H | H |
| 19 | 4-Cl-Ph | 5-Pyrimi | 3-Cl-Ph | H | H | H |
| 20 | 2,4-Cl₂-Ph | 5-Pyrimi | 4-Cl-Ph | H | H | H |
| 21 | 2,4-Cl₂-Ph | 5-Pyrimi | 2-Cl-Ph | H | H | H |
| 22 | 2,4-Cl₂-Ph | 5-Pyrimi | 3-Cl-Ph | H | H | H |
| 23 | 4-Cl-Ph | 5-Pyrimi | 2,4-Cl₂-Ph | H | H | H |
| 24 | 2-Cl-Ph | 5-Pyrimi | 2,4-Cl₂-Ph | H | H | H |
| 25 | 3-Cl-Ph | 5-Pyrimi | 2,4-Cl₂-Ph | H | H | H |
| 26 | 2-F,4-Cl-Ph | 5-Pyrimi | 4-Cl-Ph | H | H | H |
| 27 | 2-F,4-Cl-Ph | 5-Pyrimi | 2-Cl-Ph | H | H | H |
| 28 | 2-F,4-Cl-Ph | 5-Pyrimi | 3-Cl-Ph | H | H | H |
| 29 | 2-F,4-Cl-Ph | 3-Py | 4-Cl-Ph | H | H | H |
| 30 | 2-F,4-Cl-Ph | 3-Py | 2-Cl-Ph | H | H | H |
| 31 | 2-F,4-Cl-Ph | 3-Py | 3-Cl-Ph | H | H | H |
| 32 | 2,4-F₂-Ph | 5-Pyrimi | 4-Cl-Ph | H | H | H |
| 33 | 2,4-F₂-Ph | 5-Pyrimi | 2-Cl-Ph | H | H | H |
| 34 | 2,4-F₂-Ph | 5-Pyrimi | 3-Cl-Ph | H | H | H |
| 35 | 4-Cl-Ph | 4-F,3-Py | 2,4-Cl₂-Ph | H | H | H |
| 36 | 2-Cl-Ph | 4-Me,3-Py | 2,4-Cl₂-Ph | H | H | H |
| 37 | 3-Cl-Ph | 5-MeO,3-Py | 2,4-Cl₂-Ph | H | H | H |
| 38 | 4-Cl-Ph | 3-Py | 2,4-Cl₂-Ph | C(O)-c-Prop | H | H |
| 39 | 2-Cl-Ph | 3-Py | 2,4-Cl₂-Ph | H | H | Ph |
| 40 | 3-Cl-Ph | 3-Py | 2,4-Cl₂-Ph | H | Me | Me |
| 41 | 4-Cl-Ph | 4-Me,5-Pyrimi | 4-Cl-Ph | H | H | H |
| 42 | 4-Cl-Ph | 4-MeO,5-Pyrimi | 2-Cl-Ph | H | H | H |
| 43 | 4-Cl-Ph | 5-Pyrimi | 3-CF₃-Ph | H | H | H |
| 44 | 2-Thioph | 3-Py | 4-Cl-Ph | H | H | H |
| 45 | 4-Cl-Ph | 3-Py | 2-Thioph | H | H | H |
| 46 | 2-Fur | 3-Py | 2,4-Cl₂-Ph | H | H | H |
| 47 | 2,4-Cl₂-Ph | 3-Py | 2-Fur | H | H | H |
| 48 | 3-Fur | 3-Py | 2,4-Cl₂-Ph | H | H | H |
| 49 | 2,4-Cl₂-Ph | 3-Py | 3-Fur | H | H | H |
| 50 | c-Hx | 3-Py | 4-Cl-Ph | H | H | H |
| 51 | c-Hx | 3-Py | 2,4-F₂-Ph | H | H | H |
| 52 | 2-Cl-Ph | 3-Py | c-Hx | H | H | H |
| 53 | 2,4-Cl₂-Ph | 3-Py | c-Hx | H | H | H |
| 54 | i-Prop | 3-Py | 4-Cl-Ph | H | H | H |
| 55 | 2,4-Cl₂-Ph | 3-Py | i-Prop | H | H | H |
| 56 | 2,4-Cl₂-Ph | 3-Py | i-Amyl | H | H | H |
| 57 | 4-Cl-Ph | 3-Py | Et | H | H | H |
| 58 | 2,4-Cl₂-Ph | 3-Py | 4-Cl-Ph | H | CN | H |
| 59 | 2,4-Cl₂-Ph | 3-Py | 4-Cl-Ph | C(O)Me | CN | H |
| 60 | Bn | 3-Py | 4-Cl-Ph | H | CN | H |
| 61 | 2,4-Cl₂-Ph | 3-Py | 4-Cl-Ph | H | H | OMe |

In the above table, the following is meant by each abbreviation given for R¹ to R⁶:

| | | | |
|---|---|---|---|
| H | Hydrogen | 2-Fur | 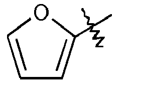 |
| CN | 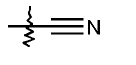 | 3-Fur | 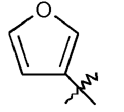 |
| Me | 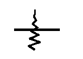 | 2-Thioph | 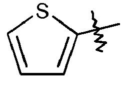 |
| Et | 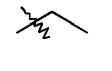 | 2-Cl-Ph | 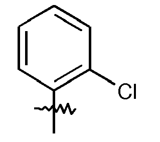 |
| Bu | 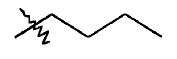 | 3-Cl-Ph | 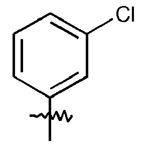 |
| i-Prop | 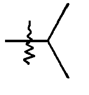 | 4-Cl-Ph | 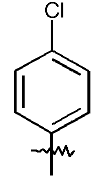 |
| i-Amyl | 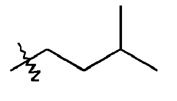 | 2,4-Cl₂-Ph | 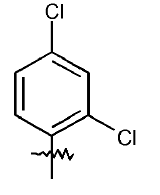 |
| OMe | 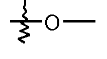 | 2-F,4-Cl-Ph | 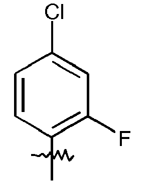 |
| C(O)Me | 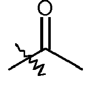 | 2,4-F₂-Ph | 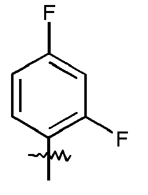 |
| C(O)Et | 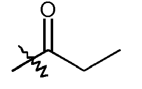 | 3-CF₃-Ph | 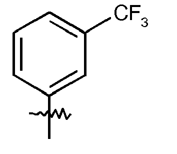 |
| C(O)-c-Prop | 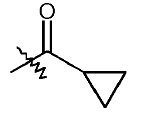 | 4-F,3-Py | 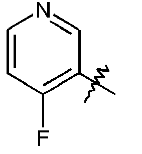 |
| c-Hx | 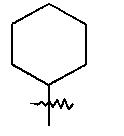 | 4-Me,3-Py | 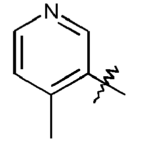 |
| Bn | 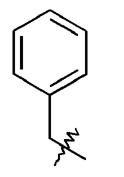 | 5-MeO,3-Py | 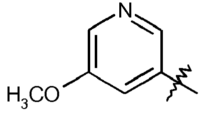 |
| Ph | 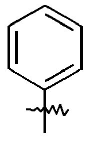 | 4-Me,5-Pyrimi | 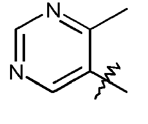 |
| 3-Py | 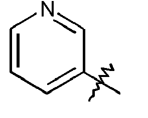 | 4-MeO,5-Pyrimi | 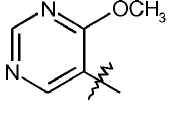 |
| 5-Pyrimi | 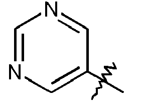 | | |

Compounds of the invention and for use in the methods of the invention can be made, for example, by following the reaction scheme and the methods detailed below. The starting materials used for the preparation of the compounds of the invention may be purchased from usual commercial suppliers or may be prepared by known methods. The starting materials as well as the intermediates may be purified before use in the next step by state of the art methodologies such as chromatography, crystallization, distillation and filtration.

N-substituted pyrroles can easily be prepared according to Acta Chemica Scandinavia, 1952, 6, 867

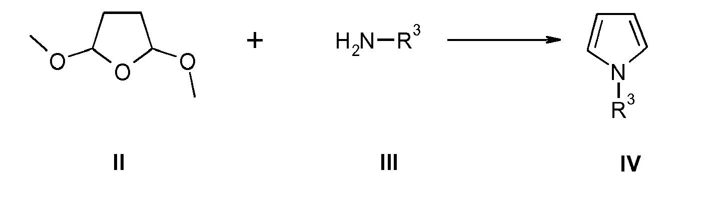

Bromination of IV with NBS will provide V according to Journal of Organic Chemistry, 1990, 55, 6317 or Tetrahedron, 1990, 31, 6785

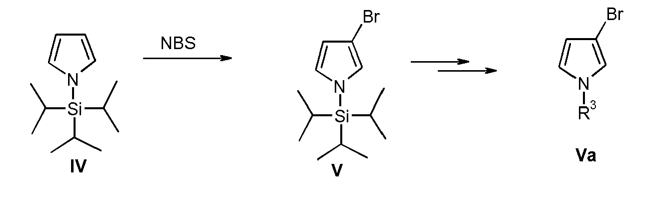

Metallation of the intermediate V in position 2 followed by trapping with an aldehyde will lead to compounds of type VI

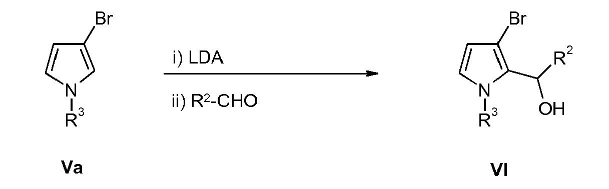

Conversion of VI to final compounds can be achieved following a procedure given in Synthesis, 2006, 22, 3883 or according to methods described in "Palladium in Heterocyclic Chemistry", Jie Jack Li and Gordon W. Gribble; Pergamon 2000.

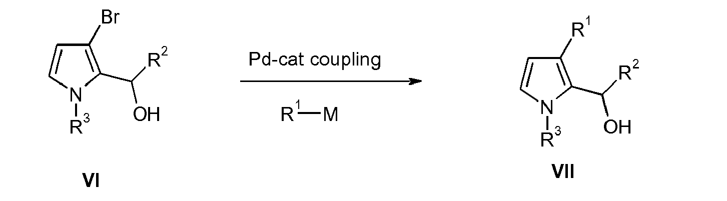

In Synthesis, 2006, 22, 3883 methods are described to prepare also 1-, 2-, 3- and 4-substituted pyrroles of generic formula I:

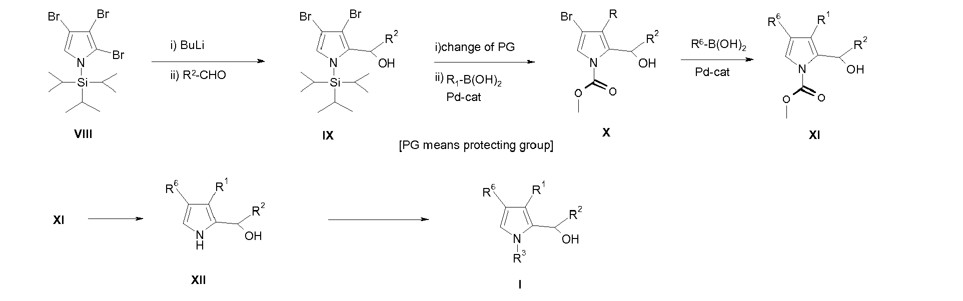

Alternative methods for the preparation of 1-, 2- and 3-substituted pyrroles can be found in Tetrahedron, 1996, 52, 6879

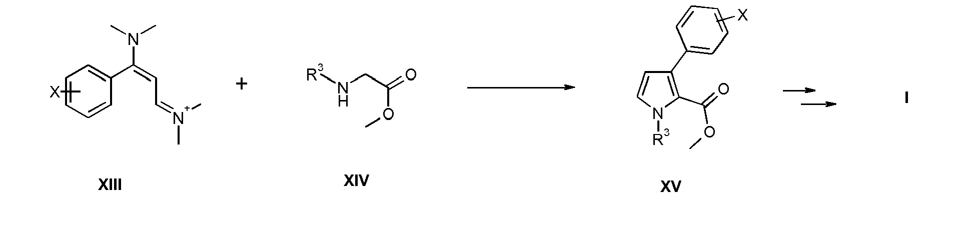

The compounds of the present invention are useful in controlling plant pathogenic fungi when they are applied to a plant or plant propagation material or the locus thereof in a fungicidally effective amount. Accordingly, therefore, the present invention also provides a method of preventing and/or controlling fungal infection in plants and/or plant propagation material comprising applying to the plant or plant propagation material or the locus thereof a fungicidally effective amount of a compound of formula I.

By 'plant propagation material' is meant generative parts of a plant including seeds of all kinds (fruit, tubers, bulbs, grains etc), roots, rhizomes, cuttings, cut shoots and the like. Plant propagation material may also include plants and young plants which are to be transplanted after germination or after emergence from the soil.

By "locus" is meant the fields on which the plants to be treated are growing, or where the seeds of cultivated plants are sown, or the place where the seed will be placed into the soil.

The compounds of the present invention may be used against phytopathogenic fungi of the following classes: Fungi imperfecti (e.g. Botrytis, Pyricularia, Helminthosporium, Fusarium, Septoria, Cercospora and Alternaria), Basidiomycetes (e.g. Rhizoctonia, Hemileia, Puccinia), Ascomycetes (e.g. Venturia and Erysiphe, Podosphaera, Monilinia, Uncinula and Pyrenophora) and Oomycetes (e.g. Phytophthora, Pythium, Plasmopara). In particular, the compounds of the present invention may be used against *Helminthosporium* spp., *Fusarium* spp., *Septoria* spp., *Cercospora* spp., *Alternaria* spp., *Rhizoctonia* spp., *Puccinia* spp., *Venturia* spp., *Erysiphe* spp., *Podosphaera* spp., *Monilinia* spp., *Uncinula* spp. and *Pyrenophora* spp.

The compounds of the present invention are suitable for controlling fungal disease on a number of plants and their propagation material including, but not limited to the following target crops: cereals (wheat, barley, rye, oats, maize (including field corn, pop corn and sweet corn), rice, sorghum and related crops); beet (sugar beet and fodder beet); leguminous plants (beans, lentils, peas, soybeans); oil plants (rape, mustard, sunflowers); cucumber plants (marrows, cucumbers, melons); fibre plants (cotton, flax, hemp, jute); vegetables (spinach, lettuce, asparagus, cabbages, carrots, eggplants, onions, pepper, tomatoes, potatoes, paprika, okra); plantation crops (bananas, fruit trees, rubber trees, tree nurseries), ornamentals (flowers, shrubs, broadleaved trees and evergreens, such as conifers); as well as other plants such as vines, bushberries (such as blueberries), caneberries, cranberries, peppermint, rhubarb, spearmint, sugar cane and turf grasses including, but not limited to, cool-season turf grasses (for example, bluegrasses (*Poa L.*), such as Kentucky bluegrass (*Poa pratensis* L.), rough bluegrass (*Poa trivialis* L.), Canada bluegrass (*Poa compressa* L.) and annual bluegrass (*Poa annua* L.); bentgrasses (*Agrostis* L.), such as creeping bentgrass (*Agrostis palustris* Huds.), colonial bentgrass (*Agrostis tenius* Sibth.), velvet bentgrass (*Agrostis canina* L.) and redtop (*Agrostis alba* L.); fescues (*Festuca* L.), such as tall fescue (*Festuca arundinacea* Schreb.), meadow fescue (*Festuca elatior* L.) and fine fescues such as creeping red fescue (*Festuca rubra* L.), chewings fescue (*Festuca rubra* var. commutata Gaud.), sheep fescue (*Festuca ovina* L.) and hard fescue (*Festuca longifolia*); and ryegrasses (*Lolium* L.), such as perennial ryegrass (*Lolium perenne* L.) and annual (Italian) ryegrass (*Lolium multiflorum* Lam.)) and warm-season turf grasses (for example, Bermudagrasses (*Cynodon* L. C. Rich), including hybrid and common Bermudagrass; Zoysiagrasses (*Zoysia Willd*.), St. Augustinegrass (*Stenotaphrum secundatum* (Walt.) Kuntze); and centipedegrass (*Eremochloa ophiuroides* (Munro.) Hack.)).

In addition 'crops' are to be understood to include those crops that have been made tolerant to pests and pesticides, including herbicides or classes of herbicides, as a result of conventional methods of breeding or genetic engineering. Tolerance to e.g. herbicides means a reduced susceptibility to damage caused by a particular herbicide compared to conventional crop breeds. Crops can be modified or bred so as to be tolerant, for example, to HPPD inhibitors such as mesotrione or EPSPS inhibitors such as glyphosate.

The compounds of formula I may be in unmodified form or, preferably, may be incorporated into fungicidal compositions. Typically the compounds of formula I are therefore formulated together with carriers and adjuvants conventionally employed in the art of formulation, using methods well known to the person skilled in the field of formulation.

The invention therefore also relates to a composition for the control of fungal infection comprising a compound of formula I and an agriculturally acceptable carrier or diluent.

The invention further relates to a composition for the control of fungal infection comprising a compound of formula I, an agriculturally acceptable carrier or diluent and at least one additional fungicide .

The agrochemical composition will usually contain from 0.1 to 99% by weight, preferably from 0.1 to 95% by weight, of the compound of formula I, 99.9 to 1% by weight, preferably 99.8 to 5% by weight, of a solid or liquid adjuvant, and from 0 to 25% by weight, preferably from 0.1 to 25% by weight, of a surfactant.

Suitably, the agrochemical compositions of the present invention are applied prior to disease development. Rates and frequency of use of the formulations are those conventionally used in the art and will depend on the risk of infestation by the fungal pathogen, the developmental stage of the plant and on the location, timing and application method. Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient rates of application are from 10mg to 1g of active substance per kg of seeds.

In practice, as indicated above, the agrochemical compositions comprising compound of formula I are applied as a formulation containing the various adjuvants and carriers known to or used in the industry. They may thus be formulated as granules, as wettable or soluble powders, as emulsifiable concentrates, as coatable pastes, as dusts, as flowables, as solutions, as suspensions or emulsions, or as controlled release forms such as microcapsules. These formulations are described in more detail below and may contain as little as about 0.5% to as much as about 95% or more by weight of the active ingredient. The optimum amount will depend on formulation, application equipment and nature of the plant pathogenic fungi to be controlled.

Suspension concentrates are aqueous formulations in which finely divided solid particles of the active compound are suspended. Such formulations include antisettling agents and dispersing agents and may further include a wetting agent to enhance activity as well an anti-foam and a crystal growth inhibitor. In use, these concentrates are diluted in water and normally applied as a spray to the area to be treated. The amount of active ingredient may range from about 0.5% to about 95% of the concentrate.

Wettable powders are in the form of finely divided particles which disperse readily in water or other liquid carriers. The particles contain the active ingredient retained in a solid matrix. Typical solid matrices include fuller's earth, kaolin clays, silicas and other readily wet organic or inorganic solids. Wettable powders normally contain about 5% to about 95% of the active ingredient plus a small amount of wetting, dispersing or emulsifying agent.

Emulsifiable concentrates are homogeneous liquid compositions dispersible in water or other liquid and may consist entirely of the active compound with a liquid or solid emulsifying agent, or may also contain a liquid carrier, such as xylene, heavy aromatic naphthas, isophorone and other non-volatile organic solvents. In use, these concentrates are dispersed in water or other liquid and normally applied as a spray to the area to be treated. The amount of active ingredient may range from about 0.5% to about 95% of the concentrate.

Granular formulations include both extrudates and relatively coarse particles and are usually applied without dilution to the area in which control of plant pathogenic fungi is required. Typical carriers for granular formulations include sand, fuller's earth, attapulgite clay, bentonite clays, montmorillonite clay, vermiculite, perlite, calcium carbonate, brick, pumice, pyrophyllite, kaolin, dolomite, plaster, wood flour, ground corn cobs, ground peanut hulls, sugars, sodium chloride, sodium sulphate, sodium silicate, sodium borate, magnesia, mica, iron oxide, zinc oxide, titanium oxide, antimony oxide, cryolite, gypsum, diatomaceous earth, calcium sulphate and other organic or inorganic materials which absorb or which can be coated with the active compound. Granular formulations normally contain about 5% to about 25% active ingredients which may include surface-active agents such as heavy aromatic naphthas, kerosene and other petroleum fractions, or vegetable oils; and/or stickers such as dextrins, glue or synthetic resins.

Dusts are free-flowing admixtures of the active ingredient with finely divided solids such as talc, clays, flours and other organic and inorganic solids which act as dispersants and carriers.

Microcapsules are typically droplets or granules of the active ingredient enclosed in an inert porous shell which allows escape of the enclosed material to the surroundings at controlled rates. Encapsulated droplets are typically about 1 to 50 microns in diameter. The enclosed liquid typically constitutes about 50 to 95% of the weight of the capsule and may include solvent in addition to the active compound. Encapsulated granules are generally porous granules with porous membranes sealing the granule pore openings, retaining the active species in liquid form inside the granule pores. Granules typically range from 1 millimetre to 1 centimetre and preferably 1 to 2 millimetres in diameter. Granules are formed by extrusion, agglomeration or prilling, or are naturally occurring. Examples of such materials are vermiculite, sintered clay, kaolin, attapulgite clay, sawdust and granular carbon. Shell or membrane materials include natural and synthetic rubbers, cellulosic materials, styrene-butadiene copolymers, polyacrylonitriles, polyacrylates, polyesters, polyamides, polyureas, polyurethanes and starch xanthates.

Other useful formulations for agrochemical applications include simple solutions of the active ingredient in a solvent in which it is completely soluble at the desired concentration, such as acetone, alkylated naphthalenes, xylene and other organic solvents. Pressurised sprayers, wherein the active ingredient is dispersed in finely-divided form as a result of vaporisation of a low boiling dispersant solvent carrier, may also be used.

Suitable agricultural adjuvants and carriers that are useful in formulating the compositions of the invention in the formulation types described above are well known to those skilled in the art. Suitable examples of the different classes are found in the non-limiting list below.

Liquid carriers that can be employed include water, toluene, xylene, petroleum naphtha, crop oil, acetone, methyl ethyl ketone, cyclohexanone, acetic anhydride, acetonitrile, acetophenone, amyl acetate, 2-butanone, chlorobenzene, cyclohexane, cyclohexanol, alkyl acetates, diacetonalcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, N,N-dimethyl formamide, dimethyl sulfoxide, 1,4-dioxane, dipropylene glycol, dipropylene glycol methyl ether, dipropylene glycol dibenzoate, diproxitol, alkyl pyrrolidinone, ethyl acetate, 2-ethyl hexanol, ethylene carbonate, 1,1,1-trichloroethane, 2-heptanone, alpha pinene, d-limonene, ethylene glycol, ethylene glycol butyl ether, ethylene glycol methyl ether, gamma-butyrolactone, glycerol, glycerol diacetate, glycerol monoacetate, glycerol triacetate, hexadecane, hexylene glycol, isoamyl acetate, isobornyl acetate, isooctane, isophorone, isopropyl benzene, isopropyl myristate, lactic acid, laurylamine, mesityl oxide, methoxy-propanol, methyl isoamyl ketone, methyl isobutyl ketone, methyl laurate, methyl octanoate, methyl oleate, methylene chloride, m-xylene, n-hexane, n-octylamine, octadecanoic acid, octyl amine acetate, oleic acid, oleylamine, o-xylene, phenol, polyethylene glycol (PEG400), propionic acid, propylene glycol, propylene glycol monomethyl ether, p-xylene, toluene, triethyl phosphate, triethylene glycol, xylene sulfonic acid, paraffin, mineral oil, trichloroethylene, perchloroethylene, ethyl acetate, amyl acetate, butyl acetate, methanol, ethanol, isopropanol, and higher molecular weight alcohols such as amyl alcohol, tetrahydrofurfuryl alcohol, hexanol, octanol, etc. ethylene glycol, propylene glycol, glycerine, N-methyl-2-pyrrolidinone, and the like. Water is generally the carrier of choice for the dilution of concentrates.

Suitable solid carriers include talc, titanium dioxide, pyrophyllite clay, silica, attapulgite clay, kieselguhr, chalk, diatomaxeous earth, lime, calcium carbonate, bentonite clay, fuller's earth, cotton seed hulls, wheat flour, soybean flour, pumice, wood flour, walnut shell flour, lignin and the like.

A broad range of surface-active agents are advantageously employed in both said liquid and solid compositions, especially those designed to be diluted with carrier before application. These agents, when used, normally comprise from 0.1% to 15% by weight of the formulation. They can be anionic, cationic, non-ionic or polymeric in character and can be employed as emulsifying agents, wetting agents, suspending agents or for other purposes. Typical surface active agents include salts of alkyl sulfates, such as diethanolammonium lauryl sulphate; alkylarylsulfonate salts, such as calcium dodecylbenzenesulfonate; alkylphenol-alkylene oxide addition products, such as nonylphenol-C.sub. 18 ethoxylate; alcohol-alkylene oxide addition products, such as tridecyl alcohol-C.sub. 16 ethoxylate; soaps, such as sodium stearate; alkytnaphthalenesulfonate salts, such as sodium dibutylnaphthalenesulfonate; dialkyl esters of sulfosuccinate salts, such as sodium di(2-ethylhexyl) sulfosuccinate; sorbitol esters, such as sorbitol oleate; quaternary amines, such as lauryl trimethylammonium chloride; polyethylene glycol esters of fatty acids, such as polyethylene glycol stearate; block copolymers of ethylene oxide and propylene oxide; and salts of mono and dialkyl phosphate esters.

Other adjuvants commonly utilized in agricultural compositions include crystallisation inhibitors, viscosity modifiers, suspending agents, spray droplet modifiers, pigments, antioxidants, foaming agents, anti-foaming agents, light-blocking agents, compatibilizing agents, antifoam agents, sequestering agents, neutralising agents and buffers, corrosion inhibitors, dyes, odorants, spreading agents, penetration aids, micronutrients, emollients, lubricants, sticking agents, and the like.

In addition, further, other biocidally active ingredients or compositions may be combined with the compound of formula I and used in the methods of the invention and applied simultaneously or sequentially with the compound of formula I. When applied simultaneously, these further active ingredients may be formulated together with the compound of formula I or mixed in, for example, the spray tank. These further biocidally active ingredients may be fungicides, herbicides, insecticides, bactericides, acaricides, nematicides and/or plant growth regulators. Accordingly, the present invention provides a composition comprising (i) a compound of formula I and a further fungicide, (ii) a compound of formula I and a herbicide, (iii) a compound of formula I and an insecticide, (iv) a compound of formula I and a bactericide; (v) a compound of formula I and an acaricide, (vi) a compound of formula I and a nematicide and/or (vii) a compound of formula I and a plant growth regulator. In addition, the compounds of the invention may also be applied with one or more systemically acquired resistance inducers ("SAR" inducer). SAR inducers are known and described in, for example, US Patent No. 6,919,298 and include, for example, salicylates and the commercial SAR inducer acibenzolar-S-methyl.

In particular, composition encompassed by the present invention include, but are not limited to, compositions comprising a compound of formula I and acibenzolar (CGA245704), a compound of formula I and ancymidol, a compound of formula I and alanycarb, a compound of formula I and aldimorph, a compound of formula I and amisulbrom, a compound of formula I and anilazine, a compound of formula I and azaconazole, a compound of formula I and azoxystrobin, a compound of formula I and benalaxyl, a compound of formula I and benthiavalicarb, a compound of formula I and benomyl, a compound of formula I and biloxazol, a compound of formula I and bitertanol, a compound of formula I and bixafen, a compound of formula I and blasticidin S, a compound of formula I and boscalid, a compound of formula I and bromuconazole, a compound of formula I and bupirimate, a compound of formula I and captafol, a compound of formula I and captan, a compound of formula I and carbendazim, a compound of formula I and carbendazim, a compound of formula I and chlorhydrate, a compound of formula I and carboxin, a compound of formula I and carpropamid, a compound of formula I and carvone, a compound of formula I and CGA41396, a compound of formula I and CGA41397, a compound of formula I and chinomethionate, a compound of formula I and chloroneb, a compound of formula I and chlorothalonil, a compound of formula I and chlorozolinate, a compound of formula I and clozylacon, a compound of formula I and copper containing compounds such as copper oxychloride, copper oxyquinolate, copper sulphate, copper tallate and Bordeaux mixture, a compound of formula I and cyflufenamid, a compound of formula I and cymoxanil, a compound of formula I and cyproconazole, a compound of formula I and cyprodinil, a compound of formula I and debacarb, a compound of formula I and di-2-pyridyl disulphide 1,1'-dioxide, a compound of formula I and dichlofluanid, a compound of formula I and diclomezine, a compound of formula I and dichlozoline, a compound of formula I and dichlone, a compound of formula I and dicloran, a compound of formula I and diclocymet, a compound of formula I and diethofencarb, a compound of formula I and difenoconazole, a compound of formula I and difenzoquat, a compound of formula I and diflumetorim, a compound of formula I and *O*,*O*-di-*iso*-propyl-*S*-benzyl thiophosphate, a compound of formula I and dimefluazole, a compound of formula I and dimetconazole, a compound of formula I and dimethomorph, a compound of formula I and dimethirimol, a compound of formula I and dimoxystrobin, a compound of formula I and diniconazole, a compound of formula I and dinocap, a compound of formula I and dithianon, a compound of formula I and dodecyl dimethyl ammonium chloride, a compound of formula I and dodemorph, a compound of formula I and dodine, a compound of formula I and doguadine, a compound of formula I and edifenphos, a compound of formula I and enestrobin, a compound of formula I and epoxiconazole, a compound of formula I and ethaboxam, a compound of formula I and ethirimol, a compound of formula I and etridiazole, a compound of formula I and famoxadone, a compound of formula I and fenamidone (RPA407213), a compound of formula I and fenarimol, a compound of formula I and fenbuconazole, a compound of formula I and fenfuram, a compound of formula I and fenhexamid (KBR2738), a compound of formula I and fenoxanil, a compound of formula I and fenpiclonil, a compound of formula I and fenpropidin, a compound of formula I and fenpropimorph, a compound of formula I and fentin acetate, a compound of formula I and fentin hydroxide, a compound of formula I and ferbam, a compound of formula I and ferimzone, a compound of formula I and fluazinam, a compound of formula I and fluopicolide, a compound of formula I and fludioxonil, a compound of formula I and fluoxastrobin, a compound of formula I and flumetover, a compound of formula I and SYP-LI90 (flumorph), a compound of formula I and fluopyram, a compound of formula I and fluoroimide, a compound of formula I and fluquinconazole, a compound of formula I and flusilazole, a compound of formula I and flusulfamide, a compound of formula I and flutolanil, a compound of formula I and flutriafol, a compound of formula I and folpet, a compound of formula I and fosetyl-aluminium, a compound of formula I and fuberidazole, a compound of formula I and furalaxyl, a compound of formula I and furametpyr, a compound of formula I and guazatine, a compound of formula I and hexaconazole, a compound of formula I and hydroxyisoxazole, a compound of formula I and hymexazole, a compound of formula I and IKF-916 (cyazofamid), a compound of formula I and imazalil, a compound of formula I and imibenconazole, a compound of formula I and iminoctadine, a compound of formula I and iminoctadine triacetate, a compound of formula I and ipconazole, a compound of formula I and iprobenfos, a compound of formula I and iprodione, a compound of formula I and iprovalicarb (SZX0722), a compound of formula I and isopropanyl butyl carbamate, a compound of formula I and isoprothiolane, a compound of formula I and kasugamycin, a compound of formula I and kresoxim-methyl, a compound of formula I and LY186054, a compound of formula I and LY211795, a compound of formula I and LY248908, a compound of formula I and maneb, a compound of formula I and mancopper, a compound of formula I and mancozeb, a compound of formula I and mandipropamid, a compound of formula I and mefenoxam, a compound of formula I and mepanipyrim, a compound of formula I and mepronil, a compound of formula I and metalaxyl, a compound of formula I and metconazole, a compound of formula I and methasulfocarb, a compound of formula I and metiram, a compound of formula I and metiram-zinc, a compound of formula I and metominostrobin, a compound of formula I and metrafenone, a compound of formula I and myclobutanil, a compound of formula I and myclozoline, a compound of formula I and neoasozin, a compound of formula I and nickel dimethyldithiocarbamate, a compound of formula I and nitrothal-*iso*propyl, a compound of formula I and nuarimol, a compound of formula I and ofurace, a compound of formula I and organomercury compounds, a compound of formula I and orysastrobin, a compound of formula I and oxadixyl, a compound of formula I and oxasulfuron, a compound of formula I and oxine-copper, a compound of formula I and oxolinic acid, a compound of formula I and oxpoconazole, a compound of formula I and oxycarboxin, a compound of formula I and pefurazoate, a compound of formula I and penconazole, a compound of formula I and pencycuron, a compound of formula I and penthiopyrad, a compound of formula I and phenazin oxide, a compound of formula I and phosdiphen, a compound of formula I and phosphorus acids, a compound of formula I and phthalide, a compound of formula I and picoxystrobin (ZA1963), a compound of formula I and polyoxin D, a compound of formula I and polyram, a compound of formula I and probenazole, a compound of formula I and prochloraz, a compound of formula I and procymidone, a compound of formula I and propamocarb, a compound of formula I and propiconazole, a compound of formula I and propineb, a compound of formula I and propionic acid, a compound of formula I and proquinazid, a compound of formula I and prothioconazole, a compound of formula I and pyraclostrobin, a compound of formula I and pyrazophos, a compound of formula I and pyribencarb, a compound of formula I and pyrifenox, a compound of formula I and pyrimethanil, a compound of formula I and pyroquilon, a compound of formula I and pyroxyfur, a compound of formula I and pyrrolnitrin, a compound of formula I and quaternary ammonium compounds, a compound of formula I and quinomethionate, a compound of formula I and quinoxyfen, a compound of formula I and quintozene, a compound of formula I and silthiofam, a compound of formula I and simeconazole, a compound of formula I and sipconazole (F-155), a compound of formula I and sodium pentachlorophenate, a compound of formula I and spiroxamine, a compound of formula I and streptomycin, a compound of formula I and sulphur, a compound of formula I and schwefel, a compound of formula I and tebuconazole, a compound of formula I and tecloftalam, a compound of formula I and tecnazene, a compound of formula I and tetraconazole, a compound of formula I and thiabendazole, a compound of formula I and thifluzamid, a compound of formula I and 2-(thiocyanomethylthio)benzothiazole, a compound of formula I and thiophanate-methyl, a compound of formula I and thiram, a compound of formula I and tiadinil, a compound of formula I and timibenconazole, a compound of formula I and tolclofos-methyl, a compound of formula I and tolylfluanid, a compound of formula I and triadimefon, a compound of formula I and triadimenol, a compound of formula I and triazbutil, a compound of formula I and triazoxide, a compound of formula I and tricyclazole, a compound of formula I and tridemorph, a compound of formula I and trifloxystrobin (CGA279202), a compound of formula I and triforine, a compound of formula I and triflumizole, a compound of formula I and triticonazole, a compound of formula I and validamycin A, a compound of formula I and vapam, a compound of formula I and valiphenal a compound of formula I and vinclozolin, a compound of formula I and zineb, a compound of formula I and ziram, a compound of formula I and zoxamide, a compound of formula I and 3-[5-(4-chlorophenyl)-2,3-dimethylisoxazolidin-3-yl]pyridine, a compound of formula I and 5-chloro-7-(4-methylpiperidine-1-yl)-6-(2,4,6-trifluorophenyl)[1,2,4]triazolo[1,5-a]pyrimidine and a compound of formula I and N-(4-chloro-2-nitrophenyl)-N-ethyl-4-methyl-benzsulfonamide.

The formulations of the invention and for use in the methods of the invention can be applied to the areas where control is desired by conventional methods such as spraying, atomising, dusting, scattering, coating or pouring. Dust and liquid compositions, for example, can be applied by the use of power-dusters, broom and hand sprayers and spray dusters. The formulations can also be applied from airplanes as a dust or a spray or by rope wick applications. A preferred method of applying the formulation of the invention is foliar application. In addition, both solid and liquid formulations may also be applied to the soil in the locus of the plant to be treated allowing the active ingredient to penetrate the plant through the roots. The formulations of the invention may also be used for dressing applications on plant propagation material to provide protection against fungus infections on the plant propagation material as well as against phytopathogenic fungi occurring in the soil. Suitably, the active ingredient may be applied to plant propagation material to be protected by impregnating the plant propagation material, in particular, seeds, either with a liquid formulation of the fungicide or coating it with a solid formulation. In special cases, other types of application are also possible, for example, the specific treatment of plant cuttings or twigs serving propagation. It is noted that, whereas it is preferred to formulate commercial products as concentrates, the end user will normally use dilute formulations.

Furthermore, the compounds of formula I find general use as fungicides and may therefore also be used in methods to control pathogenic fungi in related areas, for example in the protection of technical materials, in food storage or in hygiene management. As such, the present invention further provides the use of a compound of formula I for preventing and/or controlling fungal infection on technical materials, in food storage or in hygiene management. In addition, the present invention also provides a method for controlling and/or preventing infestation of technical materials by fungi comprising applying the compound of formula I to the technical material or the locus thereof in a fungicidally effective amount.

"Technical materials" include but are not limited to organic and inorganic materials such as wood, paper, leather, natural and synthetic fibers, composites thereof such as particle board, plywood, wall-board and the like, woven and nonwoven fabrics, construction surfaces and materials (e.g. building material), cooling and heating system surfaces and materials, ventilation and air conditioning system surfaces and materials, and the like. The compounds and combinations according the present invention can be applied to such materials or surfaces in an amount effective to inhibit or prevent disadvantageous effects such as decay, discoloration or mold in like manner as described above. Structures and dwellings constructed using or incorporating technical materials in which such compounds or combinations have been applied are likewise protected against attack by fungi

The technical material can be treated with a compound of formula I in a number of ways, including, but not limited to, by including the compound in the technical material itself, absorbing, impregnating, treating (in closed pressure or vacuum systems) said material with said compound, dipping or soaking the building material, or coating the material for example by curtain coating, roller, brush, spray, atomisation, dusting, scattering or pouring application. The compound of the invention can be formulated for use in treatment of technical materials by using techniques well known to the person skilled in the art. Such formulations may utilise, for example, the formulation materials listed above in relation to agrochemical formulations.

In addition to the foregoing, active compounds of the present invention can be used in the treatment of fungal infections of human and animal subjects (including but not limited to horses, cattle, sheep, dogs, cats, etc.) for medical and veterinary purposes. When the compounds of the present invention are administered as pharmaceuticals, to humans and animals, they can be given *per se* or, more typically as a pharmaceutical composition.

Accordingly, the present invention therefore provides a composition comprising a compound of formula I and a pharmaceutically acceptable carrier or diluent. In addition, the present invention also provides a method of treating fungal infection in a subject in need thereof, comprising administering a compound of formula I to said subject in an amount effective to treat said fungal infection. Furthermore, the present invention provides the use of a compound of formula I as a pharmaceutical and in a method for the manufacture of a medicament for the treatment of fungal infection.

Examples of such infections include but are not limited to ailments such as Onychomycosis, sporotichosis, hoof rot, jungle rot, *Pseudallescheria boydii,* scopulariopsis or athletes foot, sometimes generally referred to as "white-line" disease, as well as fungal infections in immunocompromised patients such as AIDS patients and transplant patients. Thus, fungal infections may be of skin or of keratinaceous material such as hair, hooves, or nails, as well as systemic infections such as those caused by *Candida* spp., *Cryptococcus neoformans,* and *Aspergillus* spp., such as in pulmonary aspergillosis and *Pneumocystis carinii* pneumonia.

"Pharmaceutically acceptable" means compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically-acceptable carrier" as used herein means a pharmaceutically-acceptable material, composition or vehicle, such as a liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Compositions of the present invention for pharmaceutical use include those suitable for oral, nasal, topical (including buccal and sublingual), rectal, vaginal and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, the particular mode of administration but will generally be that amount of the active ingredient which produces a therapeutic effect. Generally, this amount will range from about 1% to about 99% of active ingredient, preferably from about 5% to about 70%, most preferably from about 10% to about 30%.

Pharmaceutical compositions of the invention for topical, nasal, rectal and vaginal use are generally in the form of ointments, pastes, creams, gels powders and sprays. Ointments, pastes, creams and gels may contain, in addition to the compound of formula I, excipients, such as animal and vegetable fats, oils, waxes, paraffins, starch, tragacanth, cellulose derivatives, polyethylene glycols, silicones, bentonites, silicic acid, talc and zinc oxide, or mixtures thereof. Powders and sprays can contain, in addition to a compound of formula I, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

In the addition, the pharmaceutical compositions of the invention may also be suitable for oral administration. As such, they may be presented in discrete units, such as capsules, cachets, lozenges, or tablets, each containing a predetermined amount of the compound of formula I; as a powder or granules; as a solution or a suspension in an aqueous or non-aqueous liquid; or as an oil-in-water or water-in-oil emulsion.

Pharmaceutical compositions of this invention suitable for parenteral administration comprise a compound of formula I in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants. These compositions may also contain adjuvants such as preservatives, wetting agents, emulsifying agents and dispersing agents. Prevention of the action of microorganisms may be ensured by the inclusion of various antibacterial and other antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The pharmaceutical compositions of the present invention may be given by any suitable means of administration including orally, parenterally, topically, transdermally, rectally, etc. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Topical or parenteral administration is preferred.

"Parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

Actual dosage levels of the active ingredients in the pharmaceutical compositions of this invention may be varied so as to obtain an amount of the active ingredient which is effective to achieve the desired therapeutic response, e.g., antimycotic activity, for a particular patient, composition, and mode of administration, without being toxic to the patient. The selected dosage level will depend upon a variety of factors including the activity of the particular active compound employed, the route of administration, the time of administration, the rate of excretion of the particular active compound being employed, the duration of the treatment, other drugs, compounds and/or materials used in combination with the particular inhibitor employed, the age, sex, weight, condition, general health and prior medical history of the patient being treated, and like factors well known in the medical arts. A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved. As a general proposition, a dosage from about 0.01 or 0.1 to about 50, 100 or 200 mg/kg will have therapeutic efficacy, with all weights being calculated based upon the weight of the active compound, including the cases where a salt is employed.

Although the invention has been described with reference to preferred embodiments and examples thereof, the scope of the present invention is not limited only to those described embodiments. As will be apparent to persons skilled in the art, modifications and adaptations to the above-described invention can be made without departing from the spirit and scope of the invention, which is defined and circumscribed by the appended claims. All publications cited herein are hereby incorporated by reference in their entirety for all purposes to the same extent as if each individual publication are specifically and individually indicated to be so incorporated by reference.

The invention is illustrated by the following Examples:
Mass spectra data are obtained using LCMS: LC5: 254nM - gradient 10% A to 100% B; A=H₂O+0.01%HCOOH; B=CH₃CN/CH₃OH+0.01%HCOOH positive electrospray 150-1000 m/z.

### Example 1

This example illustrates the preparation of [1,3-Bis-(3-chloro-phenyl)-1H-pyrrol-2-yl]-pyridin-3-yl-methanol

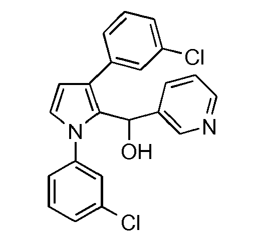

The title compound is prepared according to the following procedures:
Step A: 3-Chloroaniline (10.6 ml) and 2,5-dimethoxytetrahydrofuran (12.9 ml) are heated at 110°C in acetic acid (100 ml) for 5 hours and 30 minutes. To the cooled reaction mixture, water (500 ml) is added and the reaction is stirred overnight. The brown precipitate formed is filtered and subsequently dissolved in dichloromethane, washed with aqueous sodium hydrogencarbonate (saturated) (100 ml), dried over sodium sulphate and concentrated *in vacuo.* 1-(3-Chloro-phenyl)-1H-pyrrole is isolated as black solid (35.2 g). 1H-NMR (400 MHz, CDCl₃): 6.38 (t, 2H), 7.10 (t, 2H), 7.30 (m, 4H).
Step B: The compound obtained in Step A (24.9 g) is dissolved in *N, N-*dimethylformamide (280 ml). The reaction mixture is cooled at -67°C, and *N-*bromosuccinimide (24.9 g) is added portionwise. After 1 hour, the temperature is raised to -10 °C and the reaction stirred for further 60 minutes. The reaction mixture is partitioned between water (500 ml) and cyclohexane (300 ml) and after separation, the organic phase is washed with aqueous sodium hydrogencarbonate (saturated) (70 ml). The aqueous layer is extracted three times with cyclohexane (3 x 200 ml). The combined organic extracts are dried over sodium sulphate, concentrated *in vacuo* to obtain a brown oil (27.2 g) containing a mixture of 1-(3-Chloro-phenyl)-1H-pyrrole, 2-Bromo-1-(3-chloro-phenyl)-1H-pyrrole and 2,5-Dibromo-1-(3-chloro-phenyl)-1H-pyrrole in ratio 15:75:10, respectively. The mixture is used directly in the next step without further purification.
Step C: A solution of the crude mixture from Step B (27.2 g) and *p-*toluenesulfonic acid (2.0 g) in dichloromethane (200 ml) is stirred at room temperature for 3 hours. The reaction mixture is poured in an aqueous solution of sodium bicarbonate (saturated) (50 ml). Separation is performed and the organic phase is dried over sodium sulphate, concentrated *in vacuo* to afford 3-Bromo-1-(3-chloro-phenyl)-1H-pyrrole as red oil (25.6 g). 1H-NMR (400 MHz, CDCl₃): 6.38 (m, 1H), 6.98 (m, 1H), 7.09 (m, 1H), 7.27 (m, 1H), 7.38 (m, 3H).
Step D: A solution of the compound obtained in Step C (10.3 g) in THF (20 ml) is slowly added to a solution of freshly prepared lithium 2,2,6,6-tetramethylpiperidide (1.2 equivalents) in tetrahydrofuran (80 ml), keeping the internal temperature below -70°C. The reaction mixture is stirred at -78°C for 2 hours before adding *N*,*N*-dimethylformamide (9.3 ml). After further 2 hours at this temperature, the solution is warmed up to 0°C and quenched by addition of an aqueous solution of ammonium chloride (saturated) (70 ml). Aqueous workup with ethyl acetate furnished a crude material which is purified by chromatography on silica gel (eluent: cyclohexane/ ethylacetate = 6:1). 3-Bromo-1-(3-chloro-phenyl)-1H-pyrrole-2-carbaldehyde is isolated as a pale yellow powder (4.7 g). MS (ES+) 286/288 (MH+); 1H-NMR (400 MHz, CDCl₃): 6.49 (d, 1H), 6.99 (m, 1H), 7.24 (m, 1H), 7.34 (m, 1H), 7.43 (m, 2H), 9.76 (s, 1H).
Step E: A suspension containing 0.29 g of the compound obtained from Step D, 3-chlorophenylboronic acid (0.39 g), barium hydroxide octahydrate (0.79 g) and [1,1'-bis(diphenylphosphino)-ferrocene]dichloropalladium (II) (1/1 complex with dichloromethane, 0.082 g) in a mixture of *N*,*N*-dimethylformamide and water (4/1, 5.0 ml, 0.2 M) is quickly warmed up to 110°C with a preheated oil bath (120°C). The reaction mixture is stirred at this temperature for 1 hour, and then cooled to room temperature. Water (20 ml) and ethyl acetate (20 ml) are added. The biphasic mixture is filtered over a short pad of Hyflo eluting with ethyl acetate. The filtrate is collected and the two layers separated. The organic phase is dried over sodium sulphate and evaporated *in vacuo* to afford a brown solid which is purified by column chromatography on silica gel (eluent: cyclohexane/ ethylacetate = 6:1). 1,3-Bis-(3-chloro-phenyl)-1H-pyrrole-2-carbaldehyde (0.30 g) is isolated as a yellowish powder. MS (ES+) 316/318 (MH+); 1H-NMR (400 MHz, CDCl₃): 6.51 (m, 1H), 7.08 (m, 1H), 7.30 (m, 1H), 7.41 (m, 6H), 7.55 (m, 1H), 9.67 (s, 1H).
Step F:
   Method A: *n*-Butyllithium (0.15 ml, 2.5 M in hexane) is added dropwise to a solution of 3-bromopyridine (0.04 ml) in diethyl ether (2.0 ml) at -78°C. After 15 min of stirring at this temperature, a solution of the compound obtained in Step E (0.10 g) in tetrahydrofuran (2 ml) is added. The reaction is stirred for 30 minutes and quenched by addition of an aqueous solution of ammonium chloride (saturated) (10 ml). The mixture is extracted with ethyl acetate (3 x 10 ml). The combined organic extracts are dried over sodium sulphate and concentrated *in vacuo.* The residue is purified by column chromatography on silica gel (eluent: cylohexane/ ethylacetate =1:1) to afford the title compound, [1,3-Bis-(3-chloro-phenyl)-1H-pyrrol-2-yl]-pyridin-3-yl-methanol, as a pale yellow foam (0.090 g). MS (ES+) 395/397 (MH+); 1H-NMR (400 MHz, CDCl₃): 2.77 (bs, 1H), 6.03 (s, 1H), 6.31 (d, 1H), 6.69 (d, 1H), 6.89 (m, 1H), 7.00 (m, 1H), 7.14 (m, 7H), 7.38 (m, 1H), 8.05 (m, 1H), 8.17 (m, 1H).
   Method B: Equimolecular amount (1.2 eq) of 3-bromopyridine and *i-*propylmagnesium chloride (2.0 M in tetrahydrofuran) are stirred in tetrahydrofuran (0.1 M) at 0°C for 15 minutes and subsequently 10 minutes at room temperature. A tetrahydrofuran solution (0.1 M) of the compound isolated in Step E (1.0 eq) is added to the above slurry solution. The disappearance of the starting material is followed by TLC (eluent: cyclohexane/ ethyl acetate = 3:1). The crude reaction mixture is treated as described above.

Compounds of formula (X) (Table 2) and compounds of formula (XI) (Table3) are prepared according to procedures analogous to those described in Example 1.

**Table 2:**

| | | | | | | | |
|---|---|---|---|---|---|---|---|
| Compounds of formula (X): 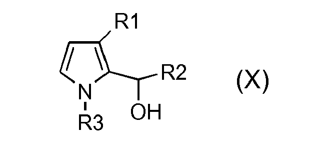 | | | | | | | |

| No | Corresponding N° in Table I | R¹ | R² | R³ | M.p. | LC-MS (RT) | MS(ES+) |
|---|---|---|---|---|---|---|---|
| 62 | - | 3-Cl-Ph | 3-Py | 3-Cl-Ph | foam | 1.69 min | 395/397 |
| 63 | - | 2-Cl-Ph | 3-Py | 3-Cl-Ph | foam | 1.07 min | 395.1 |
| 64 | - | 4-CF₃-Ph | 3-Py | 3-Cl-Ph | foam | 1.30 min | 429.1 |
| 65 | 7 | 4-Cl-Ph | 3-Py | 3-Cl-Ph | foam | 1.18 min | 395.1 |
| 66 | - | 2-OMe-Ph | 3-Py | 3-Cl-Ph | foam | 0.96 min | 391.1 |
| 67 | - | 4-OMe-Ph | 3-Py | 3-Cl-Ph | foam | 1.02 min | 391.1 |
| 68 | - | 4-F-Ph | 3-Py | 3-Cl-Ph | foam | 1.06 min | 379.1 |
| 69 | - | 2-Me-Ph | 3-Py | 3-Cl-Ph | foam | 1.04 min | 375.1 |
| 70 | - | 4-Me-Ph | 3-Py | 3-Cl-Ph | foam | 1.11 min | 375.1 |
| 71 | - | 3-thioph | 3-Py | 3-Cl-Ph | foam | 0.92 min | 367.1 |
| 72 | - | 2-thioph | 3-Py | 3-Cl-Ph | foam | 0.96 min | 367.1 |
| 73 | - | 4-Br-Ph | 3-Py | 3-Cl-Ph | foam | 1.22 min | 439 |
| 74 | 13 | 2,4-Cl₂-Ph | 3-Py | 3-Cl-Ph | foam | 1.27 min | 429 |
| 75 | - | 2-F-Ph | 3-Py | 3-Cl-Ph | foam | 1.00 min | 379.1 |
| 76 | - | 2,4-F₂-Ph | 3-Py | 3-Cl-Ph | foam | 1.06 min | 397.1 |
| 77 | - | 3-CF₃-Ph | 3-Py | 3-Cl-Ph | foam | 1.29 min | 429.1 |
| 78 | - | 2-Cl-3-Py | 3-Py | 3-Cl-Ph | foam | 0.80 min | 396.1 |
| 79 | - | 5-Cl-2-thioph | 3-Py | 3-Cl-Ph | foam | 1.21 min | 401.0 |
| 80 | - | 2-Cl-4-OMe-Ph | 3-Py | 3-Cl-Ph | foam | 1.13 min | 425.1 |
| 81 | - | 2,4-Me₂-Ph | 3-Py | 3-Cl-Ph | foam | 1.16 min | 389.1 |
| 82 | - | 4-OCF₃-Ph | 3-Py | 3-Cl-Ph | foam | 1.33 min | 445.1 |
| 83 | - | 2-Me-4-Cl-Ph | 3-Py | 3-Cl-Ph | foam | 1.24 min | 409.1 |
| 84 | 31 | 2-F-4-Cl-Ph | 3-Py | 3-Cl-Ph | foam | 1.22 min | 413.0 |
| 85 | - | 2-Cl-4-F-Ph | 3-Py | 3-Cl-Ph | foam | 1.18 min | 413.0 |

In the above table, the following is meant by each abbreviation given for R¹ to R³:

| | | | |
|---|---|---|---|
| 2-Cl-Ph | 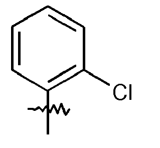 | 2-Me-Ph | 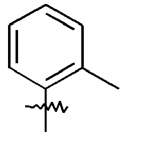 |
| 3-Cl-Ph | 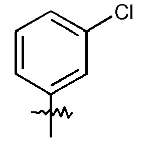 | 4-Me-Ph | 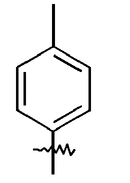 |
| 4-Cl-Ph | 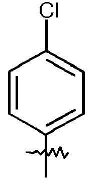 | 3-thioph | 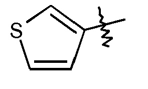 |
| 2-thioph | 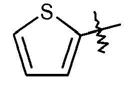 | 2-OMe-Ph | 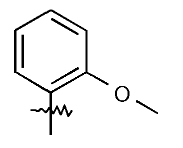 |
| 4-OMe-Ph | 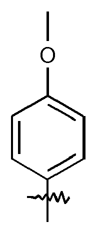 | 4-Br-Ph | 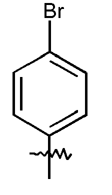 |
| 2,4-F₂-Ph | 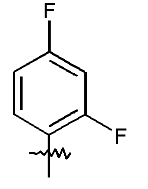 | 2,4-Cl₂-Ph | 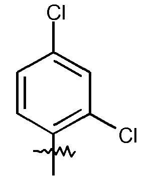 |
| 4-F-Ph | 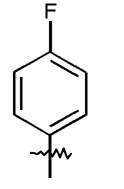 | 2-F-Ph | 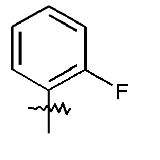 |
| 3-CF₃-Ph | 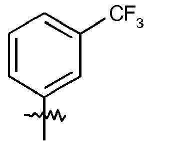 | 4-CF₃-Ph | 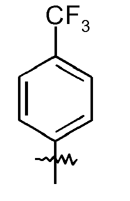 |
| 5-Cl-2-thioph | 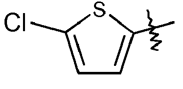 | 2-Cl-3-Py | 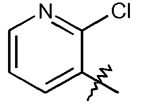 |
| 2-Cl-4-OMe-Ph | 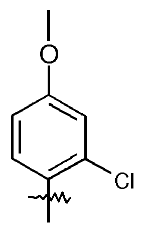 | 2,4-Me₂-Ph | 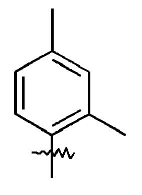 |
| 2-Me-4-Cl-Ph | 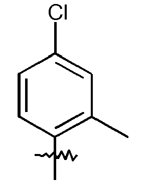 | 4-OCF₃-Ph | 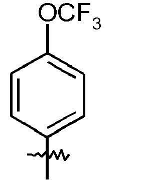 |
| 2-Cl-4-F-Ph | 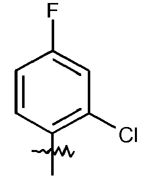 | 2-F-4-Cl-Ph | 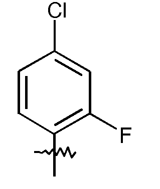 |
| 3-Py | 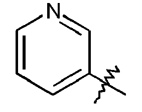 | | |

**Table 3:**

| | | | | | | |
|---|---|---|---|---|---|---|
| Compounds of formula (XI): 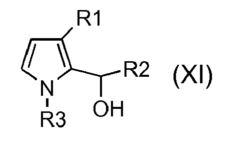 | | | | | | |

| No | R¹ | R² | R³ | M.p. | LC-MS (RT) | MS(ES+) |
|---|---|---|---|---|---|---|
| 86 | n-Prop | 3-Py | 3-Cl-Ph | foam | 1.54 min | 327/329 |

In the above table, the following is meant by each abbreviation given for R¹ to R³:

| | | | |
|---|---|---|---|
| n-Prop | 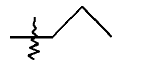 | 3-Py | 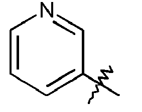 |
| 3-Cl-Ph | 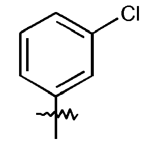 | | |

### Biological examples

This example illustrates the fungicidal properties of compounds of formula (X). The tests are performed as follows:
***Botrytis cinerea* (Gray mould):** Conidia of the fungus from cryogenic storage are directly mixed into nutrient broth (PDB potato dextrose broth). After placing a (DMSO) solution of the test compounds into a microtiter plate (96-well format) the nutrient broth containing the fungal spores is added. The test plates are incubated at 24°C and the inhibition of growth is determined photometrically after 72 hours. The following compounds give at least 80% control of *Botrytis cinerea* at 20 ppm:
   63, 64, 65, 66, 67, 68, 69, 70, 71, 72, 73, 74, 75, 76, 77, 79, 80, 82, 84 and 85.
***Mycosphaerella arachidis* (syn. *Cercospora arachidicola*), Brown leaf spot of groundnut (peanut):** Conidia of the fungus from cryogenic storage are directly mixed into nutrient broth (PDB potato dextrose broth). After placing a (DMSO) solution of the test compounds into a microtiter plate (96-well format) the nutrient broth containing the fungal spores is added. The test plates are incubated at 24°C and the inhibition of growth is determined photometrically after 72 hours at 620nm. The following compounds give at least 80% control of *Mycosphaerella arachidis* at 20 ppm:
   62, 63, 64, 65, 66, 67, 68, 69, 70, 71, 72, 73, 74, 75, 76, 77, 78, 79, 80, 81, 82, 83, 84 and 85.
***Septoria tritici* (leaf blotch):** Conidia of the fungus from cryogenic storage are directly mixed into nutrient broth (PDB potato dextrose broth). After placing a (DMSO) solution of the test compounds into a microtiter plate (96-well format) the nutrient broth containing the fungal spores is added. The test plates are incubated at 24°C and the inhibition of growth is determined photometrically after 72 hours. The following compounds give at least 80% control of *Septoria tritici* at 20 ppm:
   62, 63, 64, 65, 67, 68, 69, 70, 71, 72, 73, 74, 75, 76, 77, 79, 80, 81, 82, 83, 84 and 85.
***Monographella nivalis* (syn. *Microdochium nivale, Fusarium nivale*), snow mould, foot rot of cereals:** Conidia of the fungus from cryogenic storage are directly mixed into nutrient broth (PDB potato dextrose broth). After placing a (DMSO) solution of the test compounds into a microtiter plate (96-well format) the nutrient broth containing the fungal spores is added. The test plates are incubated at 24°C and the inhibition of growth is determined photometrically after 72 hours at 620nm. The following compounds give at least 80% control of *Monographella nivalis* at 20 ppm:
   62, 63, 64, 65, 66, 67, 68, 69, 70, 71, 72, 73, 74, 75, 76, 77, 78, 79, 80, 81, 82, 83, 84 and 85.
***Fusarium culmorum* (root rot):** Conidia of the fungus from cryogenic storage are directly mixed into nutrient broth (PDB potato dextrose broth). After placing a (DMSO) solution of the test compounds into a microtiter plate (96-well format) the nutrient broth containing the fungal spores is added. The test plates are incubated at 24°C and the inhibition of growth is determined photometrically after 48 hours. The following compounds give at least 80% control of *Fusarium culmorum* at 20 ppm:
   63 and 85.

## Claims

1. A compound of formula I: 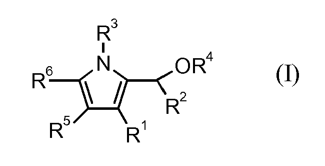 R¹ and R³ are, independently, hydrogen, or optionally substituted alkyl, alkenyl, alkynyl, heterocyclyl, trialkylsilyl, arylalkyl, aryloxyalkyl, arylthioalkyl, aryl or heteroaryl, with the proviso that they are not both hydrogen;
R² is optionally substituted heterocyclyl or heteroaryl;
R⁴ is H or acyl, haloacyl, alkoxycarbonyl, aryloxycarbonyl, alkylaminocarbonyl or dialkylaminocarbonyl group;
R⁵ and R⁶ are, independently, hydrogen, cyano, halogen or optionally substituted alkyl, alkenyl, alkynyl, heterocyclyl, alkoxy, alkoxycarbonyl, alkylthio, trialkylsilyl, arylalkyl, aryloxyalkyl, arylthioalkyl, aryl or heteroaryl;
or a salt or N-oxide thereof.

2. The compound of claim 1, wherein R¹ and R³ are, independently, hydrogen or optionally substituted alkyl, alkenyl, alkynyl, heterocyclyl or trialkylsilyl.

3. The compound of claim 1, R¹ and R³ are, independently, hydrogen or optionally substituted alkyl, aryloxyalkyl, arylthioalkyl, aryl or heteroaryl.

4. The compound of claim 1, wherein R¹ and R³ are, independently, hydrogen or optionally substituted alkyl, phenyl or 5- or 6-membered heteroaryl.

5. The compound of claim 4, wherein R² is optionally substituted pyridyl, pyrimidinyl or thiazolyl.

6. The compound of any one of claims 1 to 5, wherein R⁴ is H.

7. The compound of any one of claims 1 to 6, wherein R⁵ and R⁶ are, independently hydrogen, cyano or halogen or optionally substituted alkyl, alkenyl, alkynyl, alkoxy, alkylthio, trialkylsilyl or alkoxycarbonyl.

8. The compound of claim 7, wherein R⁵ and R⁶ are, independently hydrogen, cyano, halogen, alkyl, alkoxy or alkylthio.

9. The compound of any one of claims 1 to 8, wherein at least one of R¹, R³, R⁵, and R⁶ is not hydrogen.

10. The compound of claim 1, wherein R¹ and R³ are, independently, optionally substituted aryl or heteroaryl;

11. R² is optionally substituted heteroaryl; and
R⁴, R⁵, and R⁶ are hydrogen.

12. The compound of claim 10, wherein R¹ and R³ are, independently, optionally substituted phenyl, thienyl, pyridyl or furyl;
R² is optionally substituted pyridyl or pyrimidinyl; and
R⁴, R⁵, and R⁶ are hydrogen.

13. The compound of claim 11, wherein R¹ is 2-chloro-phenyl, 3-chloro-phenyl, 4-chloro-phenyl, 4-bromo-phenyl, 2-fluoro-phenyl, 4-fluoro-phenyl, 2,4-dichloro-phenyl, 2,4-difluoro-phenyl, 2-fluoro-4-chloro-phenyl, 2-chloro-4-fluoro-phenyl, 2-methyl-phenyl, 4-methyl-phenyl, 2,4-dimethyl-phenyl, 2-methoxy-phenyl, 4-methoxy-phenyl, 3-trifluoromethyl-phenyl, 4-trifluoromethyl-phenyl, 2-chloro-4-methoxy-phenyl, 4-methoxytrifluomethyl-phenyl, 2-methyl-4-chloro-phenyl, 2-chloro-3-pyridyl, 2-thienyl, 3-thienyl or 5-chloro-2-thienyl.

14. The compound of claim 11, wherein R³ is 3-chloro-phenyl.

15. The compound of claim 11, wherein R² is 3-pyridyl.

16. The compound of claim 1, wherein R¹ is hydrogen or optionally substituted alkyl and R³ is optionally substituted aryl or heteroaryl.

17. A composition for the control of fungal infection comprising a compound of formula I as defined in any one of claims 1 to 16 and an agriculturally acceptable carrier or diluent.

18. The composition of claim 17, further comprising at least one additional fungicide.

19. A method of preventing and/or controlling fungal infection in plants and/or plant propagation material comprising applying to the plant or plant propagation material or the locus thereof a fungicidally effective amount of a compound of formula I as defined in any one of claims 1 to 16.
